# FASCICULE DE BREVET EUROPEEN

(11) **EP 1 200 000 B1**
(45) Date de publication et mention de la délivrance du brevet: **13.10.2004**
(21) Numéro de dépôt: 00949665.4
(22) Date de dépôt: 07.07.2000
(51) Int. Cl.: A61B 18/20

(54) **DISPOSITIF DE TRANSFERT CONTROLE DE RAYONNEMENT LASER POUR LA CHIRURGIE ESTHETIQUE**
GERÄT ZUR KONTROLLIERTEN LASERBESTRAHLUNG FÜR DIE SCHÖNHEITCHIRURGIE
DEVICE FOR CONTROLLED TRANSFER OF LASER RADIATION FOR COSMETIC SURGERY

(30) Priorité: 12.07.1999 FR 9909357
(43) Date de publication de la demande: 02.05.2002
(73) Titulaire: Uhlrich, Yves-Marie, 69006 Lyon (FR)
(72) Inventeur: Uhlrich, Yves-Marie, 69006 Lyon (FR)
(74) Mandataire: Schmitt, John
(86) Numéro de dépôt international: PCT/FR2000/001957
(87) Numéro de publication internationale: WO 2001/003597

(56) Documents cités:
- EP-A- 0 610 991
- WO-A-92/17138
- WO-A-93/03678
- DE-A- 19 703 208
- US-A- 4 122 853
- US-A- 5 498 260
- US-A- 5 578 029

## Description

L'invention concerne un dispositif de transfert contrôlé de rayonnement laser pour la chirurgie esthétique, notamment pour faire disparaître ou au moins atténuer les défauts de la peau du visage tels que les angiomes, les taches bénignes. les rides.

Par les brevets EP-A-0610991, WO-A-92/17138, WO-A-93/03678, US-A5498260 et US-A-4122853 on connaît des dispositifs de transfert contrôlé de rayonnement laser utilisant un rayonnement laser susceptible de se propager dans l'air et dans une fibre optique. Ces dispositifs sont destinés essentiellement à la chirurgie, c'est-à-dire qu'ils sont utilisés comme bistouri pour l'ablation de tissus et susceptibles de pénétrer le corps par les voies naturelles ou par une incision faite par un autre instrument.

On connaît aussi par le brevet US-A-5578029 un dispositif utilisé pour le traitement des veines par rayonnement laser susceptible de se propager dans une fibre optique reliée à un manche et dont l'extrémité est équipée d'une sonde creuse pointue autorisant son enfoncement dans le derme.

Le dispositif objet de l'invention ne s'applique qu'à la chirurgie esthétique dont l'une des activités vise les rides du visage qui ont un caractère permanent car elles apparaissent à la suite de la perte d'élasticité du derme qui se détend de manière permanente en formant des replis ; cette perte d'élasticité est due entre autre à la dégradation des propriétés élastiques des fibres d'un élément composant le derme qui s'appelle le collagène.

Pour compenser cette perte d'élasticité la chirurgie esthétique utilise ou a utilisé de nombreuses solutions en fonction de l'importance des rides ; certaines de ces solutions consistent à compenser les distensions en retendant la peau du visage ou à introduire une prothèse pour combler les vides créés par la diminution de la quantité de collagène, telle qu'un fil d'une matière plastique souple d'un diamètre de l'ordre d'un millimètre qu'on place sous le derme en suivant le trajet de la ride, chacune de ces solutions étant choisie spécifiquement en fonction de la nature et de l'importance du problème à résoudre ; une autre solution consiste à introduire du collagène sous l'épiderme ce qui est une solution efficace à court terme, mais qui est à recommencer régulièrement.

La chirurgie esthétique utilise d'autre part l'action d'un rayonnement laser sur la peau pour un certain nombre d'interventions spécifiques. La chirurgie esthétique utilise plusieurs types de rayonnement laser en fonction de la nature de l'intervention ; un premier rayonnement laser, qui utilise du gaz carbonique et qu'on appelle ci-après «rayonnement laser long», a une longueur d'onde de l'ordre de dix microns située dans l'infrarouge lointain et a la propriété d'être absorbé par le verre et par l'eau. Un autre rayonnement laser appelé ci-après «rayonnement laser Yag» émet un rayonnement dans le spectre infrarouge proche d'une longueur d'onde de mille soixante quatre nanomètres et a la propriété de pénétrer en profondeur et d'être absorbé par les tissus ; un autre rayonnement laser qu'on appelle ci-après rayonnement Erbium a une longueur d'onde d'environ deux mille quatre-vingts nanomètres située dans l'infrarouge proche a la propriété d'être absorbé par l'eau tout en étant transportable par fibre optique. Un autre qu'on appelle «rayonnement laser Yag-doublé» a une longueur d'onde de cinq cent trente deux nanomètres a la propriété d'être absorbé par le sang. Le rayonnement laser Yag ou laser-doublé ou laser Erbium, qu'on appelle ci-après «rayonnement laser court», peut se propager aussi bien dans l'air que dans une fibre 'optique, servant de dispositif de guidage de rayonnement et peut être facilement amené à l'endroit utile.

Le rayonnement laser long dirigé sur la peau agit d'abord sur l'épiderme dont il fait éclater les cellules qui contiennent essentiellement de l'eau ; il permet notamment de faire disparaître des ridules par abrasion cutanée ; on peut utiliser dans ce cas un rayonnement laser long ultra-pulsé qui permet d'utiliser des impulsions d'une puissance de rayonnement très élevée pendant un temps de l'ordre de la microseconde avec une fréquence de l'ordre de quelques dizaines de hertz ; le rayonnement laser long ultra-pulsé a l'avantage de limiter considérablement l'inflammation du derme. Le rayonnement laser long se propage naturellement dans l'air, mais ne peut se propager dans une fibre optique ; lorsqu'il doit être dirigé sur un endroit déterminé, il faut utiliser un dispositif de guidage d'onde aérien combiné avec des miroirs appropriés.

Le rayonnement laser Yag-doublé a la propriété de traverser les cellules de l'épiderme, sans la dégrader de manière significative, pour s'attaquer aux parois des vaisseaux et au sang qu'ils contiennent, situés dans le derme ; il est utilisé notamment pour réduire les angiomes.

Une technique utilisant le rayonnement laser long ultra-pulsé permet d'une part de faire une abrasion cutanée pour effacer des ridules, par exemple au niveau de la bordure supérieure des lèvres ou des paupières inférieures.

On constate que lorsque le rayonnement laser ultra-pulsé s'approche du derme, par amincissement de l'épiderme et y provoque un effet thermique, il génère, au niveau du derme, une réaction de rétractation durable tout en réactivant la création de collagène par le derme.

La possibilité d'utiliser l'effet thermique sur le derme du rayonnement laser long pour réduire les rides reste limité parce qu'on ne peut atteindre les couches profondes du derme que par conduction thermique ; le flux thermique, qui traverse le derme, est difficile à contrôler, il peut-être à l'origine de lésions entraînant l'apparition de cicatrices visibles. Le rayonnement laser Yag ou laser Erbium peut créer un effet thermique au niveau du derme, mais il dégrade l'épiderme et il est difficile d'en contrôler la profondeur de pénétration ce qui peut engendrer des lésions notamment au niveau des tissus profonds.

L'invention est telle que définie dans la revendication 1.

L'objet de l'invention concerne un dispositif de transfert contrôlé de rayonnement laser permettant de l'amener à agir, par effet thermique, directement sur le derme, en éliminant les risques de détérioration de l'épiderme et en protégeant les tissus sous-jacents au derme. Dans ce but, le dispositif comporte une tige rigide creuse terminée à son extrémité libre par une pointe autorisant une progression sous-cutanée de ladite tige qui est d'abord enfoncée sous le derme en suivant le trajet d'une ride de manière qu'une fenêtre latérale d'où sort le rayonnement laser soit dirigée vers le derme pour en provoquer sa rétraction.

Selon l'invention et pour permettre l'interchangeabilité de l'aiguille après chaque intervention, le dispositif se caractérise par l'utilisation d'un guide d'onde en deux parties constitué d'une première fibre optique allant du générateur à un manche servant à tenir et à manoeuvrer l'aiguille sous la peau, le guide d'onde comportant une interface entre le manche et l'aiguille qui est parcourue par la deuxième partie du guide d'onde qui amène le faisceau laser dans le système optique de déviation interne à l'aiguille.

Sur les dessins annexés :
La figure 1 représente une vue perspective du dispositif de transfert contrôlé de rayonnement laser selon l'invention, avant assemblage pour mise en oeuvre d'un dispositif de guidage de rayonnement laser avec un manche relié avec une source émettrice du rayonnement.
La figure 2 représente une coupe en élévation de l'assemblage du manche avec une première extrémité du dispositif de guidage de rayonnement laser de la figure 1.
La figure 3 représente une coupe en élévation de la deuxième extrémité du dispositif de guidage de rayonnement laser.
La figure 4 représente une vue perspective partielle de la deuxième extrémité du dispositif de guidage de rayonnement laser avec une découpe d'une fenêtre latérale.
La figure 5 représente un prisme optique susceptible de s'encastrer dans la fenêtre latérale représentée figure 4.
Les figures 6 et 7 représentent une coupe partielle d'un dispositif de guidage de rayonnement laser suivant deux variantes de l'invention.

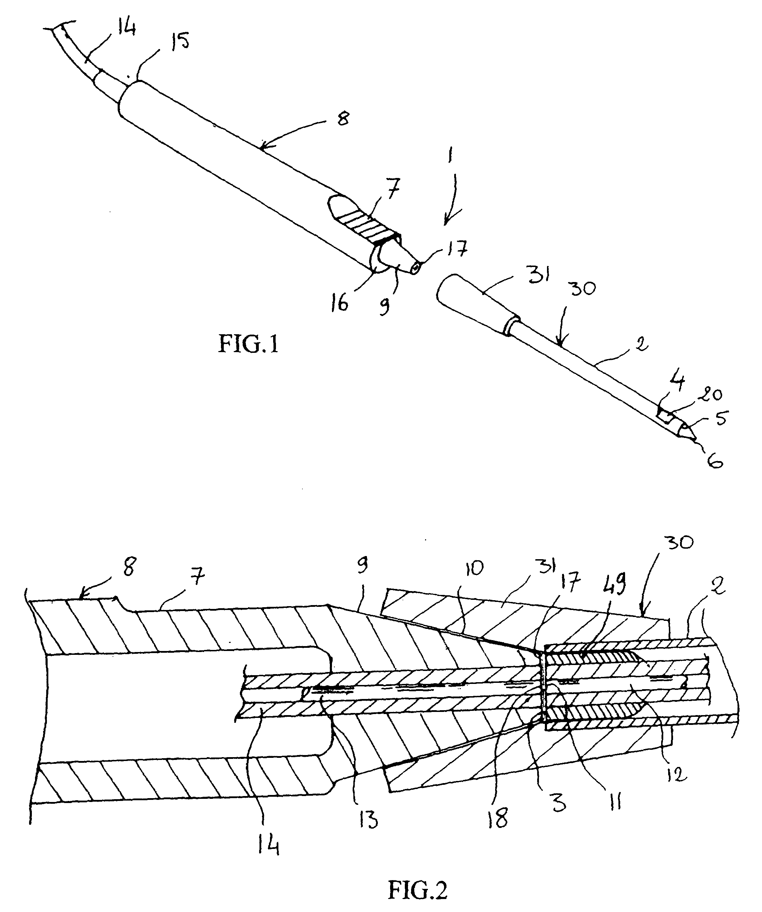

Le dispositif de transfert contrôlé de rayonnement laser 1 (fig.1) objet de l'invention, est constitué d'un dispositif de guidage de rayonnement laser 30 interchangeable associé à un manche 8 ; le dispositif de guidage de rayonnement laser 30 dont le corps principal est constitué d'une tige rigide creuse 2, contient un moyen de guidage de rayonnement laser ; ce dernier est parcouru par un rayonnement laser entrant à une première extrémité 3 (fig. 2) et ressortant, après déviation par un dispositif réfléchissant, par une fenêtre latérale 4 (fig.1) pratiquée dans la paroi de la tige creuse 2, au voisinage de la deuxième extrémité 5 ; cette dernière est terminée par une pointe 6 qui est d'une forme appropriée permettant une progression sous-cutanée de la tige rigide creuse 2. La tige rigide creuse 2 est d'abord enfoncée sous le derme ou dans le derme, en suivant le trajet d'une ride, de manière que la fenêtre latérale 4 soit dirigée vers le derme ; le rayonnement laser qui sort par la fenêtre latérale 4 agit alors directement sur le derme et provoque sa rétraction ; le chirurgien ressort progressivement la tige rigide creuse 2 tout en laissant agir le rayonnement laser par effet thermique sur les tissus tout le long de la ride à traiter : pendant toute l'opération, l'orientation de la fenêtre latérale 4 est contrôlée par un moyen de repérage extérieur solidaire d'un manche 8 sur lequel vient se fixer le dispositif de guidage de rayonnement laser 30.

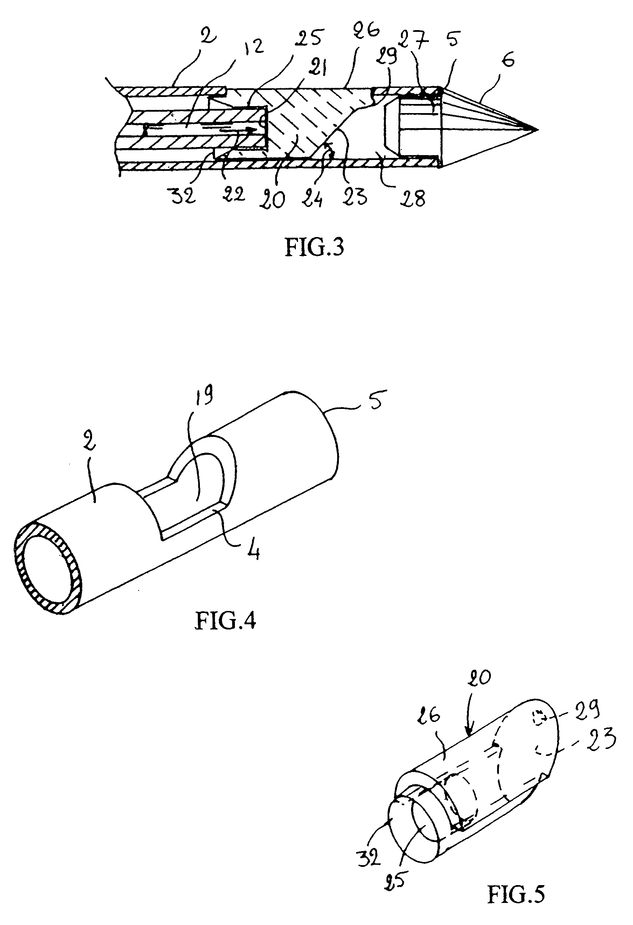

De préférence, le rayonnement laser utilisé est un rayonnement laser court susceptible d'être transporté par fibre optique du type de ceux précédemment décrits ; la tige rigide creuse 2 est un tube cylindrique de révolution métallique, comme par exemple de l'acier inoxydable ; la première extrémité 3 (fig.2) comprend par exemple un embout 31 en matière plastique surmoulée sur la tige rigide creuse 2, de même nature que celui que comportent les aiguilles ou trocarts de seringues jetables ; cet embout 31 coopère, par un moyen de fixation et de centrage de la tige rigide creuse 2, avec un contre-embout 9 solidaire du manche 8 qui sert de moyen de manoeuvre ; ce moyen de fixation et de centrage comporte, par exemple, un logement central tronconique de révolution 10 au centre du fond duquel, qui est constitué par la première extrémité 3. se trouve la première extrémité 11 d'une fibre optique 12, qui est tenue en position centrée par un manchon 49 qui va vers la deuxième extrémité 5 (fig.1 et 3) de la tige rigide creuse 2 ; le manche 8 est dimensionné pour être facilement tenu et manipulé par le chirurgien ; une fibre optique 13 (fig.2) venant d'un générateur de rayonnement laser est dans une gaine 14 (fig.1 et 2) qui traverse le manche 8 de part en part ; la fibre optique 13 entre, par exemple, par l'extrémité arrière 15 du manche 8 et ressort par l'extrémité avant 16 au niveau du contre-embout 9 ; le contre-embout 9 est constitué d'une excroissance tronconique de révolution qui vient se loger et s'ajuster dans le logement central tronconique de révolution 10 ; la fibre optique 13 débouche au centre du sommet 17 du contre-embout 9 tronconique de révolution et son extrémité 18 est située dans le plan de ce sommet 17. Selon le mode de réalisation susmentionné, l'embout 31 de la tige creuse 2 est emmanché à force sur le contre-embout 9 du manche 8, mais l'embout et le contre-embout peuvent aussi comporter un filetage conique pour permettre une meilleure fiabilité de la liaison. Dans ces conditions, l'extrémité 18 de la fibre optique 13 provenant du générateur se place juste en face de la première extrémité 11 de la fibre optique 12 située dans la tige rigide creuse 2 et le rayonnement laser peut passer d'une fibre optique à l'autre.

On décrit un embout 31 femelle solidaire de la tige rigide creuse 2 et un contre-embout 9 mâle solidaire du manche 8, mais leurs formes peuvent être inversées si la réalisation technique rend, par exemple, plus facile d'avoir un embout mâle du côté de la tige rigide creuse 2.

Au voisinage de la deuxième extrémité 5 (fig.1, 3 et 4) de la tige rigide creuse 2 se trouve la fenêtre latérale 4 communiquant largement avec la partie creuse 19 de la tige rigide creuse 2 comportant un dispositif réfléchissant déviant le flux de rayonnement laser ce dispositif réfléchissant est un prisme optique 20 (fig.3 et 5) de matière plastique transparente au rayonnement laser court ; le prisme optique 20 comporte une face d'entrée 32, une face de réflexion totale 23 et une face de sortie 26 du rayonnement laser court la deuxième extrémité 21 de la fibre optique 12 interne à la tige rigide creuse 2 pénètre dans le prisme optique 20 par la face 32 dans une direction 22 parallèle à la direction principale de la tige rigide creuse 2 par un logement cylindrique de révolution 25 d'une profondeur suffisante pour qu'elle puisse y tenir par un emmanchement à force ; le prisme optique 20 comporte une face de réflexion totale 23, par exemple plane, présentant une inclinaison faisant un angle 24 convenable avec la direction 22 de la fibre optique 12 pour obtenir une réflexion totale du rayonnement laser court et sa déviation vers la fenêtre latérale 4 ; la forme de la face de réflexion totale 23 du prisme optique 20 au niveau de la fenêtre latérale 4 peut varier en fonction du degré de concentration qu'on veut donner au flux de rayonnement laser court lorsqu'il sort de la fenêtre latérale 4. De préférence, le rayonnement laser court ressort du prisme 20 par la face de sortie 26 (fig.3 et 5) qui est située dans le prolongement de la surface extérieure de la tige creuse rigide 2 et recouvre toute la surface de la fenêtre latérale 4 de manière à obtenir une étanchéité suffisante pour empêcher l'introduction de liquide susceptible de modifier les qualités optiques du prisme 20 ; le prisme optique 20 est placé dans la fenêtre 4 après y avoir fixé la fibre optique 12 et il y est maintenu, par exemple, par une retenue 29 susceptible de se clipser avec le bord de la fenêtre latérale 4. La deuxième extrémité 5 de la tige rigide creuse 2 comporte une pointe 6 dont la forme peut varier en fonction du résultat recherché et qui comporte un tourillon 27 d'emmanchement de la pointe 6 dans la deuxième extrémité 5 de la tige rigide creuse 2 de manière à l'obturer et à délimiter, de manière étanche, un volume d'air 28, derrière la face de réflexion totale 23, afin d'éviter qu'un liquide ne vienne s'interposer et modifier les propriétés optiques du prisme optique 20.

Lorsque la tige rigide creuse 2 (fig.1) est fixée au bout du manche 8, l'orientation de la fenêtre latérale 4 doit être repérée par rapport au manche 8. Le chirurgien peut alors introduire dans le derme ou sous le derme la tige rigide creuse 2 et la retirer progressivement tout en balayant, par de petites rotations dans un sens et dans l'autre de la tige rigide creuse 2, la face interne du derme par le rayonnement laser court, en sachant la position de la fenêtre latérale 4. Le repérage de la position'du manche 8 est réalisé, par exemple, par la création d'une zone de positionnement 7, sensiblement plane servant d'appui au pouce au niveau de l'extrémité avant 16 du manche ; en cours d'opération cette zone de positionnement 7 est tenue du côté opposé à la surface de la peau à traiter par rapport au manche 8 ; lorsque l'emmanchement de la tige rigide creuse 2 sur le manche 8 est conique et se fait à force, il suffit de positionner la fenêtre latérale 4 en face de la zone de positionnement 7.

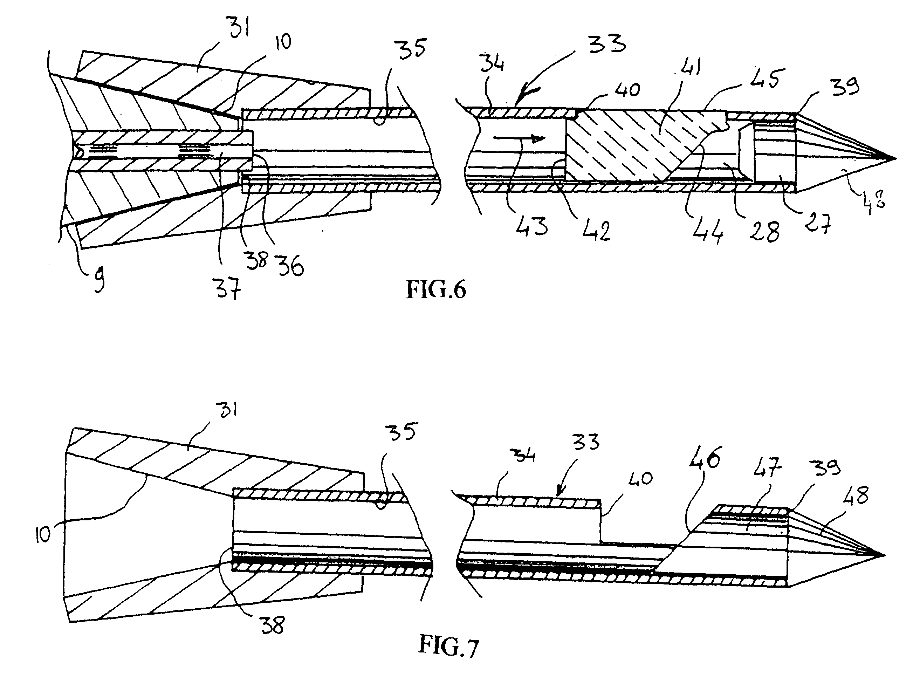

Les figures 6 et 7 montrent un dispositif de guidage de rayon laser 33 qui ne comporte pas de fibre optique interne, la tige rigide creuse 34 est métallique et de forme cylindrique de révolution et sa paroi interne 35 est polie pour obtenir la qualité d'un miroir ; si on utilise un rayonnement laser court, l'extrémité 36 (fig.6) de la fibre optique 37 pénètre de préférence légèrement dans la première extrémité 3 de la tige rigide creuse 34 ; au voisinage de la deuxième extrémité 39 de la tige rigide creuse 34 se trouve une fenêtre latérale 40 dans laquelle est placé un prisme optique 41 comprenant une face d'entrée 42, perpendiculaire à la direction principale 43 de la tige rigide creuse 34, une face de réflexion totale 44 et une face de sortie 45 du rayonnement laser court ; si on utilise un rayonnement laser long ce dernier est émis par un guide d'onde débouchant à l'extrémité du contre embout du manche 8 et traverse la tige rigide creuse 34 (fig.7) pour venir frapper un miroir métallique 46 déviant le rayonnement laser long vers la fenêtre 40 ; de préférence , c'est l'extrémité du tourillon 47 de fixation de la pointe 48 qui est conformée et polie pour faire office de miroir métallique 46. En fonction des puissances utilisées, il peut être nécessaire d'associer un dispositif d'évacuation des fumées de la tige rigide creuse 34 fonctionnant par balayage d'air acheminé vers la deuxième extrémité de la tige rigide creuse par l'intermédiaire de cette dernière et évacué par aspiration par une gaine entourant la tige rigide creuse 34.

## Revendications

1. Dispositif de transfert contrôlé de rayonnement laser (1) utilisant un rayonnement laser susceptible de se propager dans l'air et dans une fibre optique (12), ledit dispositif étant composé d'un manche (8) relié à un générateur de rayonnement laser associé à un dispositif de guidage de rayonnement laser (30,33) interchangeable, ledit manche comprenant une extrémité arrière, une extrémité avant et un moyen de repérage extérieur (7), le corps principal dudit dispositif de guidage étant une tige rigide creuse (2,34) comportant une première extrémité (3) coopérant avec l'extrémité avant du manche (8) par des moyens d'assemblage et une seconde extrémité, ladite tige creuse (2,34) comportant au voisinage de sa seconde extrémité une fenêtre latérale (4,40) pratiquée dans la paroi et associée à un dispositif réfléchissant (20,41,46), la seconde extrémité de la tige creuse (2,34) étant équipée d'une pointe (6) tronconique autorisant l'enfoncement de ladite tige creuse (2,34) sous le derme pour en permettre une progression sous-cutanée, **caractérisé en ce que** le dispositif de guidage de rayonnement laser (30) comprend par ailleurs une première partie de fibre optique (13) allant du générateur jusqu'à ladite extrémité avant du manche (8) et une seconde partie (12) séparée de la précédente incluse dans la tige creuse (2) allant de ladite première extrémité (3) jusqu'au dispositif réfléchissant (20), et **en ce que** les moyens d'assemblage sont constitués d'un embout (31), solidaire de la première extrémité de la tige rigide creuse (2), coopérant avec un contre embout (9) tronconique de révolution ayant un sommet solidaire de l'extrémité avant du manche (8), grâce à un moyen de fixation et de centrage de la tige rigide creuse (2) comportant un logement central tronconique de révolution (10) dont le fond est constitué par la première extrémité de la tige creuse (2).

2. Dispositif suivant la revendication 1 **caractérisé en ce que** ladite seconde partie (12) de fibre optique comprend une première extrémité (11) qui coopère avec l'extrémité (18) de la première partie de fibre optique, cette dernière étant située au centre du contre embout et une deuxième extrémité (21) qui coopère avec le dispositif réfléchissant (20) qui permet la déviation du rayonnement laser par la fenêtre (4).

3. Dispositif suivant la revendication 1, **caractérisé en ce que** le moyen de repérage extérieur de la position du manche (8) est réalisé par une zone de positionnement (7) servant d'appui au pouce au niveau de l'extrémité avant du manche (8).

4. Dispositif suivant la revendication 1, **caractérisé en ce que** la pointe (6) comporte un tourillon (27) d'emmanchement dans la deuxième extrémité de la tige rigide creuse (2,34) de manière à l'obturer.

5. Dispositif suivant la revendication 1, **caractérisé en ce que** l'embout (31) et le contre embout (9) tronconique de révolution comportent un filetage conique.

## Patentansprüche

1. Vorrichtung zum kontrollierten Transfer von Laserstrahlung (1) unter Einsatz eines zur Verbreitung in der Luft und in einer Lichtleitfaser (12) geeigneten Laserstrahls, wobei die genannte Vorrichtung aus einem mit einer austauschbaren Laserstrahlführvorrichtung (30, 33) zugeordneten einem Laserstrahlgenerator verbundenen Griff (8) gebildet wird, wobei der genannte Griff ein hinteres Ende, ein vorderes Ende und ein äußeres Markierungsmittel (7) umfasst, wobei der Hauptkörper der genannten Führungsvorrichtung ein hohler steifer Stift (2, 34) mit einem ersten, mit dem vorderen Ende des Griffs (8) durch Zusammenbaumittel zusammenwirkendes Ende (3) und ein zweites Ende ist, wobei der genannte hohle Stift (2, 34) in der Nähe seines zweiten Endes ein in die Wand praktiziertes und einer reflektierenden Vorrichtung (20, 41, 46) zugeordnetes laterales Fenster (4, 40) umfasst, wobei das zweite Ende des hohlen Stiftes (2, 34) mit einer kegelstumpfartigen Spitze (6) ausgerüstet ist, die das Eindrücken des genannten hohlen Stifts (2, 34) unter die Haut erlaubt, um ihre Progression unter der Haut zu erlauben, **dadurch gekennzeichnet, dass** die Laserstrahlführvorrichtung (30) darüber hinaus einen vom Generator bis zum genannten vorderen Ende des Griffs (8) gehenden ersten Lichtleitfaserteil (13) und einen vom vorherigen getrennten, im hohlen Stift (2) inbegriffenen, vom genannten ersten Ende (3) bis zur reflektierenden Vorrichtung (20) gehenden zweiten Teil (12) umfasst, und dass die Zusammenbaumittel aus einem fest mit dem ersten Ende des hohlen steifen Stiftes (2) verbundenen, mit einem kegelstumpfartigen Umdrehungsgegenansatz (9) zusammenwirkenden Ansatz (31) mit einer mit dem vorderen Ende des Griffs (8) dank eines Befestigungs- und Zentrierungsmittels des hohlen steifen, Stiftes (2) mit einer kegelstumpfartigen, zentralen Umdrehungsaufnahme (10), deren Boden durch das erste Ende des hohlen Stiftes (2) gebildet wird, umfassenden zusammenwirkenden Spitze gebildet werden.

2. Vorrichtung gemäß Anspruch 1, **dadurch gekennzeichnet, dass** der genannte zweite Teil (12) der Lichtleitfaser ein erstes Ende (11), das mit dem Ende (18) des ersten Lichtleitfaserteils zusammenwirkt, wobei letzterer sich in der Mitte des Gegenansatzes befindet, und ein zweites Ende (21) umfasst, das mit der reflektierenden Vorrichtung (20) zusammenwirkt, die die Ableitung der Laserstrahlung durch das Fenster (4) erlaubt.

3. Vorrichtung gemäß Anspruch 1, **dadurch gekennzeichnet, dass** das äußere Markierungsmittel der Position des Griffs (8) durch einen als Stütze für den Daumen auf der Höhe des vorderen Endes des Griffs (8) dienenden Positionierungsbereich (7) gebildet wird.

4. Vorrichtung gemäß Anspruch 1, **dadurch gekennzeichnet, dass** die Spitze (6) einen Zapfen (27) zum Einstecken in das zweite Ende des hohlen steifen Stiftes (2, 34) umfasst, so dass dieser verschlossen wird.

5. Vorrichtung gemäß Anspruch 1, **dadurch gekennzeichnet, dass** der Ansatz (31) und der kegelstumpfartige Umdrehungsgegenansatz (9) ein konisches Gewinde umfassen.

## Claims

1. A laser-beam control transfer device (1) using a laser beam which capable of propagating in air or in an optical fibre (12), the said device being composed of a sleeve (8) connected to a laser radiation generator, associated with an interchangeable laser-beam guidance device (30,33), the said sleeve including a back end, a front end, and a means for external location, the main body of the said guidance device being a hollow rigid rod (2,34) consisting of a first end (3) fitting onto the front end of the sleeve (8) by means of coupling devices, and a second end, the said hollow rod (2,34) having a lateral window (4,40) cut into the wall in the region of its second end, and associated with a reflecting device (20,42,46), the second end of the hollow rod (2.34) being fitted with a cone-shaped point (6) allowing the said hollow rod (2,34) to be forced under the skin in order to allow it to travel subcutaneously, **characterised in that** the laser-beam guidance device (30) also includes a first optical-fibre part (13) going from the generator to the said front end of the sleeve (8), and a second part (12), separated from the previous one, contained in the hollow rod (2) going from the said first end (3) to the reflecting device (20), and **in that** the said coupling devices are composed of a ferrule (31) joined to the first end of the hollow rigid rod (2), fitting onto a flattened cone-shaped counter-ferrule (9) whose top is joined to the front end of the sleeve (8) by virtue of a means for securing and centring the hollow rigid rod (2) having a flattened central cone-shaped housing (10) whose bottom is composed of the first end of the hollow rod (2).

2. A device according to claim 1 **characterised in that** the said second optical-fibre part (12) includes a first end (11) which fits onto the end (18) of the first optical fibre part, the latter being located at the centre of the counter-ferrule, and a second end (21) which fits onto the reflecting device (20) which is used to divert the laser beam through the window (4).

3. A device according to claim 1 **characterised in that** the external location device for the positioning of the sleeve (8) takes the form of a positioning zone (7) on which the thumb rests close to the front end of the sleeve (8).

4. A device according to claim 1 **characterised in that** the point (6) includes a cone-shaped plug (27) in the second end of the hollow rigid rod (2,34) so as to close it off.

5. A device according to claim 1 **characterised in that** ferrule (31) and the cone-shaped counter ferrule (9) include a conical thread.
